# EUROPÄISCHE PATENTSCHRIFT

(11) **EP 0 598 780 B1**
(45) Veröffentlichungstag und Bekanntmachung des Hinweises auf die Patenterteilung: **03.03.1999**
(21) Anmeldenummer: 92916955.5
(22) Anmeldetag: 12.08.1992
(51) Int. Cl.: A61B 17/39

(54) **HOCHFREQUENZCHIRURGIEGENERATOR ZUM SCHNEIDEN VON GEWEBEN**
SURGICAL HIGH-FREQUENCY GENERATOR FOR CUTTING TISSUES
GENERATEUR DE HAUTE FREQUENCE UTILE EN CHIRURGIE POUR INCISER DES TISSUS

(30) Priorität: 12.08.1991 DE 4126607; 24.10.1991 DE 4135180; 24.10.1991 DE 4135184
(43) Veröffentlichungstag der Anmeldung: 01.06.1994
(73) Patentinhaber: Karl Storz GmbH & Co., 78532 Tuttlingen (DE)
(72) Erfinder: LINDENMEIER, Heinz, D-8033 Planegg (DE); LOHR, Georg, D-8012 Ottobrunn (DE); FASTENMEIER, Karl, D-8000 München 83 (DE); FLACHENECKER, Gerhard, verstorben (DE)
(74) Vertreter: Münich, Wilhelm, Dr.
(86) Internationale Anmeldenummer: DE9200672
(87) Internationale Veröffentlichungsnummer: WO9303677

(56) Entgegenhaltungen:
- EP-A- 0 136 855
- EP-A- 0 341 446
- DE-A- 3 120 102
- DE-A- 3 530 335
- DE-A- 3 622 337

## Beschreibung

### Technisches Gebiet

Die Erfindung bezieht sich auf einen Hochfrequenz-Chirurgiegenerator für die Hochfrequenzchirurgie entsprechend dem Oberbegriff des Patentanspruchs 1.

### Stand der Technik

Ein Hochfrequenz-Chirurgiegenerator mit den Merkmalen des Oberbegriffs des Patentanspruchs 1 ist aus der DE-A-35 30 335 bekannt.

Hochfrequenzströme werden in der Chirurgie zum Schneiden vom biologischem Gewebe oder zum Koagulieren, d.h. Blutstillen verwendet. Hochfrequenzströme werden in der Chirurgie besonders dann zum Abtragen von Gewebeteilen verwendet, wenn der Operationsort zwar durch natürliche Körperöffnungen erreichbar ist, ein Skalpell aber nicht ohne Öffnung des Körpers des Patienten angesetzt werden kann:

Zum Beispiel können in der Urologie mit transurethral eingeführten Operationsinstrumenten und mit Hilfe von Hochfrequenzströmen Tumore aus der Blase abgetragen oder krankhafte Wucherungen der Prostata entfernt werden. In der Enterologie können auf ähnliche Weise z.B. Polypen von der Darmwand abgetrennt werden. Die Sonde des Operationsinstrumentes hat dabei nur solange eine Schneidwirkung, wie der den Hochfrequenzstrom liefernde Hochfrequenzgenerator aktiviert ist. Damit ist ein gefahrloses Einbringen und Entfernen des Operationsinstrumentes durch die natürlichen Körperöffnungen gewährleistet.

Einige der heute üblichen Generatoren - beispielsweise der aus der DE-A-35 30 335 bekannte Generator - sind in mindestens einer elektrischen Ausgangsgröße wie Strom, Ausgangsspannung, Leistung, Leerlaufspannung oder Innenwiderstand elektronisch einstellbar. Durch diese Einstellung kann unmittelbar oder mittelbar die Ausgangsleistung beeinflußt werden. Diese Ausgangsleistung muß entsprechend dem Operationsziel und den Bedingungen am Operationsort eingestellt werden.

Ein Problem in der Hochfrequenzchirurgie ist die richtige Dosierung der momentan applizierten Hochfrequenzleistung. Die für gute Schneidwirkung mindestens notwendige Hochfrequenzleistung kann sehr stark schwanken. Sie hängt von der Gewebebeschaffenheit, der Leitfähigkeit und dem Wassergehalt des Gewebes, der Sondenform und Sondengröße, der Schnitt-Tiefe und Schnittgeschwindigkeit und weiteren elektrischen Parametern ab, die alle im Laufe einer Operation gewissen, oft sehr abrupt auftretenden Änderungen unterworfen sind. Die übliche, aus der Erfahrung des Operateurs gewonnene Einstellung des Hochfrequenzgenerators führt daher im Mittel zu einer deutlich überhöhten Hochfrequenzleistung, deren Gefahren der Operateur seinen Patienten und sich selbst bewußt aussetzen muß. Um diese Gefahren so klein wie möglich halten zu können bzw. nahezu vollständig ausschließen zu können, müßte die momentane Ausgangsleistung des Hochfrequenzgenerators automatisch so geregelt sein, daß sie in jedem Zeitmoment dem absolut notwendigen Mindestmaß entspricht.

Ein besonderes Problem hierbei ist der Schnittbeginn. Je nach dem Zustand der Sonde und des Gewebes können die Anfangsbedingungen sehr stark variieren. So kann die Sonde metallisch blank mit einer sauberen Oberfläche und damit gut leitend sein. Ebenso kann sie nach mehreren Schnitten oder Koagulationen von einer isolierenden Oxidschicht oder karbonisierten Geweberesten überzogen sein. Um diese Schicht zu durchschlagen ist eine hohe Generatorspannung notwendig. Maßgeblich für das Durchschlagen der isolierenden Schicht ist die elektrische Feldstärke, die über der Durchschlagsfeldstärke dieser Schicht liegen muß. Der Generator muß eine entsprechend hohe Spannung abgeben.

Das Gewebe selbst kann durch vorhergehende Schnitte oder Koagulationen verändert sein. Besonders schwierig ist das Anschneiden bei Operationen an Magen und Darm. Hier ist die Gewebeoberfläche sehr feucht und elektrisch besonders gut leitend. Auch hier ist das Anschneiden nur mit einer sehr hohen Generatorleistung möglich. Wichtig hierbei ist eine hohe Leistungszufuhr und damit ein hoher Generatorstrom, um die leitfähige Flüssigkeit möglichst schnell zu verdampfen.

Ist die Leistungsabgabe des Generators konstant, so muß der Generator vom Operateur so eingestellt werden, daß er mindestens die zum Anschneiden notwendige Leistung abgibt. Wie Messungen im Labor und in Kliniken gezeigt haben, liegt diese zum Anschneiden notwendige Leistung wesentlich über der zur Fortsetzung des Schnittes notwendigen Leistung. Der Operateur wird den Schnitt nun mit einer unveränderten Einstellung fortsetzen und nimmt damit die bekannten Risiken einer zu hohen Leistungsabgabe in Kauf.

Ist mit einer bestimmten Generatoreinstellung ein Anschneiden nicht möglich, so führt die zugeführte Energie zu einer Koagulation der Gewebeoberfläche. Diese wird dadurch noch hochohmiger und ein Anschnieden an einer solchen Stelle ist kaum mehr möglich. Zumindest ist hierfür eine wesentlich höhere Leistungsabgabe des Generators notwendig.

Eine Lösung des Problems wird in der DE-C-25 04 280 beschrieben. Bei dieser Vorrichtung wird mit Hilfe einer Anzeigevorrichtung das Ausmaß des Lichtbogens zwischen der Sonde und dem zu schneidenden Gewebe festgestellt und das daraus abgeleitete elektrische Signal einer Regeleinrichtung zugeführt. Die Regeleinrichtung vergleicht dieses Signal mit dem Sollwertprogramm eines Sollwertgebers und leitet daraus eine Regelgröße ab, die die Ausgangsstromstärke des Generators so einstellt, daß die Intensität des Lichtbogens dem Sollwertprogramm folgt. Durch diese Regelung wird in jedem Moment die zum Schneiden minimal notwendige Leistung eingestellt. Allerdings ist der technische Aufwand bei der Realisierung beträchtlich. Für einfache und kostengünstige HF-Chirurgiegeneratoren ist daher ein anderer Lösungsweg notwendig.

Ein weiterer Lösungsvorschlag ist die in der DE-A-34 20 340 vorgeschlagene Einrichtung. Sie erhöht nach dem Einschalten des Generators die Leistung für eine konstante, voreingestellte Zeit. Wie Messungen im Labor und in Kliniken gezeigt haben, schwankt die zum Anschneiden notwendige Energie beträchtlich. Eine erhöhte Leistungsabgabe über eine vorgegebene Zeit wird der Praxis in den seltensten Fällen gerecht. Außerdem ist es beim Einsatz dieses Verfahrens unbedingt notwendig, daß beim Einschalten des Generators die Sonde am Gewebe anliegt, da die erhöhte Leistung unmittelbar nach dem Einschalten des Generators abgegeben wird. Wird erst kurze Zeit nach dem Einschalten des Generators das Gewebe berührt, so gibt der Generator bereits seine normale Schneidleistung ab und die Einrichtung hat keine Wirkung mehr.

Weitere Probleme, aber auch besondere Möglichkeiten egeben sich bei der Hochfrequenzchirurgie, wenn am Operationsort nicht nur homogenes Gewebe vorhanden ist, oder gar andersartige Materialien am Operationsort vorhanden sind.

So unterscheiden sich z.B. bei Prostataresektionen das auszuschälende Adenomgewebe vom Gewebe der Prostatakapsel, in das nicht geschnitten werden darf. Ein anderes Beispiel ist das Schneiden in der Nähe von großen Blutgefäßen. Hier kann ein Auftrennen oft eine schwer zu stillende Blutung nach sich ziehen. Kann das Blutgefäß erkannt werden, so läßt sich die Generatorleistung so verringern daß kein Schnitt mehr möglich ist. Mit einer anschließenden Koagulation kann das Gefäß verschlossen werden.

Berührt die Schneidelektrode während eines Schnittes Knochen, so kann dieser thermisch geschädigt werden. Gerade in der Zahnheilkunde ist die Gefahr besonders groß. Hier ist leicht ein zu tiefes Eindringen in die dünne Zahnfleischschicht und eine Berührung des Kieferknochens möglich. Daher sollten auch diese vom Generator erkannt werden.

Besonders nachhaltige negative Auswirkungen der Hochfrequenzchirurgie treten auf, wenn die Schneidelektrode metallisch leitende Teile im Körper des Patienten wie Implantaten, Schrauben, Nägel, Zahnfüllungen und -Kronen oder auch Operationsinstrumenten wie Pinzetten, Spiegel oder Schäfte von Resektionsinstrumenten berührt. Dabei fließt der HF-Strom von der Schneidelektrode über diese Metallischen Teile großflächig an das umliegende Gewebe ab. An den Übergangsstellen kann es zu großflächigen Koagulationen kommen. In der europäischen Patentanmeldung 91100442.2, veröffentlicht am 22.07.1992 in Form der EP-A-0 495 140, wird eine Vorrichtung beschrieben, die den Ausgangsstrom des Generators begrenzt. Damit können Gewebeschäden, die beim Berühren der Schneidelektrode von metallisch leitenden Teilen im Körper auftreten, manchmal verringert werden. Die Leistung, die beim Ansprechen der Strombegrenzung am Oerationsort an das Gewebe abgegeben wird, ist proportional zum Realteil der Impedanz des Überganges vom metallisch leitenden Teil zum umliegenden Gewebe. Damit ist die Wirksamkeit dieser Einrichtung umgekehrt proportional zur Impedanz des Gewebeüberganges. Bei kleinflächigen Gewebeübergängen ist die Impedanz sehr hoch und damit auch die umgesetzte Verlustleistung. Gerade diese kleinflächigen Gewebeübergänge sind nur mit geringer Leistung belastbar. So werden durch diese Schutzeinrichtung gerade die empfindlichsten Gewebestellen am wenigsten geschützt. Dieses Verfahren ist auch nur für spezielle Anwendungen geeignet, da die Gewebeimpedanz bei üblichen Schnitten ohne Metallberührung um mehr als eine Größenordnung schwanken kann. Dies ergibt sich aus der großen Schwankungsbreite der Schnittparameter wie Elektrodenquerschnitt, Eintauchtiefe der Schneidelektrode, Schnittgeschwindigkeit und durch den beim Schneiden immer zwischen Schneidelektrode und Gewebe brennenden Lichtbogen. So ist bei diesem Verfahren die Gefahr sehr groß, daß für einen zügigen Schnitt nicht genügend Strom zur Verfügung steht, und damit der Operateur behindert wird. Andererseits besteht aber auch die Gefahr, daß metallisch leitende Teile das Gewebe nur kleinflächig berühren. In diesem Falle ist trotz Strombegrenzung die umgesetzte Leistung hoch und es können als Folge ausgeprägte Verbrennungen entstehen. Das Verfahren versagt vollständig, wenn in Nachbarschaft zu dem zu schneidenden Gewebe hochohmiges Gewebe vorhanden ist, in das nicht geschnitten werden darf, oder das thermisch nicht belastet werden darf.

Ein weiteres Problem in der Hochfrequenzchirurgie ist die Störung anderer Einrichtungen und Geräte sowie die Reizung von Nerven und Muskeln des Patienten. Verschiedene Geräte werden durch die hohe Hochfrequenzleistung, die in der HF-Chirurgie eingesetzt wird, beeinflußt. So können Störungen induktiv, kapazitiv oder durch Strahlung einkoppeln. Häufig wird von HF-Chirurgiegeneratoren nicht ein schmalbandiges Signal bei der Grundfrequenz des Generators sondern durch steile Anstiegsflanken und Modulation ein breites Spektrum im Bereich von einigen Hz bis zu mehreren MHz abgegeben. Selbst bei Generatoren die primär nur auf der Grundwelle Leistung abgeben, entstehen beim Auftreten von Lichtbogen an der Sonde durch deren Nichtlinearitäten Harmonische der Generatorfrequenz. Damit werden häufig Geräte beeinflußt oder gestört, wenn diese nicht besonders störsicher ausgelegt wurden. Doch hier lassen sich im Allgemeinen durch gerätetechnische Verbesserungen der betroffenen Geräte die Störprobleme verringern . Eine andere Möglichkeit, speziell um die Auswirkungen von Störungen in Bildern von Videokameras zu minimieren ist in der europäischen Patentschrift 0429204A1 beschrieben. Darin wird das gepulste Ausgangssignal eines HF-Chirurgiegenerators auf das Signal einer Videokamera synchronisiert, so daß die durch den Generator hervorgerufenen hochfrequenten Störungen nur unwesentliche Teile des Kamerabildes beeinflussen. Durch das Austasten mit einer derart hohen Frequenz entsteht ein HF-Signal mit hohem Crestfaktor, wie es üblicherweise zur Koagulation verwendet wird. Wegen der dadurch erzeugten Koagulation ist ein nekrosearmes Schneiden nicht möglich. Ein Schneiden ohne störende Nekrose ist erst bei einer Austastung mit Frequenzen unterhalb von 1000 Hz möglich.

Ein wesentlich wichtigerer Effekt sind die Auswirkungen auf den Körper des Patienten. Obwohl die HF-Chirurgie auf den thermischen Wirkungen des Stromes basiert, sind die elektrischen Effekte nicht vernachlässigbar. Durch Signale mit den heute üblichen Generatorfrequenzen über 300kHz (nach VDE) treten keine nachweisbaren Reizungen von Muskeln und Nerven auf. Doch durch die beim Schneiden immer auftretenden Lichtbogen entsteht auch ein Anteil mit sehr niedriger Frequenz nahe 0, die 0-te Harmonische des Generatorsignals. Durch diesen Anteil, der wie Messungen gezeigt haben, durchaus mit Amplituden bis zu einigen Volt auftreten kann, sind Stimulationen von Muskeln und Nerven möglich. Diese sind jedoch im Allgemeinen unerwünscht und häufig gefährlich.

So genügt bereits eine Energie von 10 J um die Kontraktion eines Muskels auszulösen. Diese können zu plötzlichen, starken Bewegungen des Patienten auf dem Operationstisch führen. Als Folge können Verletzungen des Patienten auftreten. Wesentlich gefährlicher ist allerdings die Reizung des Herzmuskels. So können bereits kurze Impulse mit einer Energie 400 Ws zu einem Herzkammerflimmern führen. Bei diesem selbsterregten Zustand arbeitet der Herzmuskel so schnell, daß der Blutdurchsatz sehr klein wird und der Körper nicht mehr ausreichend mit Blut versorgt wird. Als Folge tritt der Tod ein. Herzkammerflimmern kann in Kliniken mit Hilfe eines Defillibrators beseitigt werden. Bei vielen Anwendungen der HF-Chirurgie wie in kleinen Arztpraxen oder bei Zahnärzten steht jedoch ein solches Gerät nicht zur Verfügung.

Zur Vermeidung dieser gefährlichen Nebeneffekte gibt es mehrere Möglichkeiten. Die einfachste ist die Vermeidung von potentiell gefährlichen Operationen wie in der Herzgegend. Ebenso wird bei potentiell gefährdeten Patienten wie den Trägern von Herzschrittmachern häufig generell auf den Einsatz HF-Chirurgischer Operationstechniken verzichtet.

Eine weitere Möglichkeit, die Gefährdung zu Verringern, wenn auch nicht auszuschließen ist die Minimierung der für die HF-Chirurgische Operation eingesetzten Leistung. Damit werden die gefährlichen niederfrequenten Ströme ebenfalls minimal. Dies ist z.B. mit einer in der DE-C-25 04 280 beschriebenen Vorrichtung möglich. Sie mißt die Intensität des Lichtbogens und führt dem Patienten nur die minimale, zur Aufrechterhaltung des zum Schneiden notwendigen Lichtbogens benötigte Energie zu.

Bei vielen Operationstechniken bietet die HF-Chirurgie so wesentliche Vorteile, daß auch der Einsatz bei potentiell gefährdeten Patienten in potentiell gefährdeten Regionen des Körpers nöglich sein sollte. So sind besonders schonende Endoskopische Eingriffe besonders wichtig bei älteren Patienten, die allerdings häufiger Herzschrittmacher tragen.

### Darstellung der Erfindung

Der Erfindung liegt die Aufgabe zugrunde, einen Hochfrequenz-Chirurgiegenerator gemäß dem Oberbegriff des Patentanspruchs 1 derart weiterzubilden, daß das "Anschneiden" und das "Halten" des Schneidvorgangs ohne unerwünschte Nebenwirkungen erfolgt, oder diese zumindest nachhaltig reduziert werden.

Diese Aufgabe wird erfindungsgemäß durch den Gegenstand des Anspruchs 1 gelöst.

Mit Hilfe einer Meßeinrichtung zur mittelbaren und/oder unmittelbaren Feststellung des Schneidens wird ein Hochfrequenzgenerator mit Regeleinrichtungen zur Einstellung der momentanen elektrischen Ausgangsgrößen von einem Sollwertgeber eingestellt. Unmittelbar nach der Aktivierung des Generators gibt der Sollwertgeber einen Sollwert für eine Generatoreinstellung vor, die zum Anschneiden besonders geeignet ist. Dies kann je nach den Impedanzverhältnissen am Operationsort eine erhöhte Generatorspannug, ein erhöhter Generatorstrom oder auch eine erhöhte Generatorleistung sein. Entsprechend der Regelcharakteristik des Generators kann auch der Sollwert für eine andere, der Leistung entsprechenden elektrischen Größe wie Spannung oder Strom vorgegeben werden. Eine Meßeinrichtung ermittelt den Beginn des Schneidens der Sonde. Die Sonde schneidet, sobald sie unter einer Trennung des Zellverbandes in das Gewebe eindringt. Im allgemeinen tritt dabei gleichzeitig ein Lichtbogen zwischen Sonde und Gewebe auf. Sobald ein Schneiden festgestellt wird, gibt der Sollwertgeber einen niedrigeren Sollwert für den Generator vor, so daß ein Schnitt ohne unerwünschte Gewebekoagulation durchgeführt werden kann. Damit ist die mittlere Leistungszufuhr während eines Schnittes deutlich geringer als bei einer konstanten Generatoreinstellung auf einem Wert, bei dem ein Anschneiden möglich ist.

Eine besonders vorteilhafte Ausführung besteht darin, daß die Meßeinrichtung zur mittelbaren und/oder unmittelbaren Feststellung des Schneidens eine Bewegung der Sonde im Gewebe ermittelt. Dies kann z.B. durch eine Wegmessung erfolgen. Sinkt die Sonde weiter als eine von einem Sollwertgeber vorgegebene Strecke in das Gewebe ein, so wird ein Schneiden signalisiert.

Ein sicheres Kennzeichen für das hochfrequenzchirurgische Schneiden ist der Lichtbogen, der zwischen der Sonde und dem Gewebe auftritt. Daher besteht eine weitere vorteilhafte Ausführung darin, daß die Meßeinrichtung (2) zur mittelbaren und/oder unmittelbaren Feststellung des Schneidens den an der Sonde auftretenden Lichtbogen ermittelt. Dies kann vorzugsweise durch eine spektrale Betrachtung des Generatorausgangssignales erfolgen. Dazu wird ein Hochfrequenzgenerator für die Hochfrequenzchirurgie mit einer Vorrichtung zur Einstellung der maximalen Ausgangsleistung gesteuert durch das Ausgangssignal einer Anzeigevorrichtung und einer Auswerteschaltung. Zur Steuerung wird die in mindestens zwei Frequenzbereichen enthaltene elektrische Leistung herangezogen. Wahlweise kann das Ausgangssignal eines Hilfsoszillators dem Leistungsoszillatorsignal additiv überlagert werden. Nun können die Mischprodukte, die durch den Lichtbogen verursacht werden, ausgewertet und detektiert werden.

Eine detaillierte Beschreibung dieser Technik erfolgt im Zusammenhang mit dem spezifischen Aufbau des Hochfrequenzgenerators zum Detektieren unterschiedlicher Materialien.

Ein weiteres Kennzeichen für den Schnittbeginn ist, wie Messungen gezeigt haben, der Anstieg der Impedanz zwischen der Sonde und dem Gewebe. Besonders beim Schneiden von Geweben in Magen und Darm ist dieser Impedanzsprung sehr stark ausgeprägt. Hier muß zuerst die gut leitende Flüssigkeits- und Schleimschicht auf der Gewebeoberfläche verdampft werden, bevor ein Schneiden in die darunterliegenden Gewebeschichten möglich ist. Daher besteht eine weitere vorteihafte Ausführung darin, daß die Meßeinrichtung zur mittelbaren und/oder unmittelbaren Feststellung des Schneidens die Impedanz zwischen der Sonde und der Neutralelektrode auswertet. Auch hierzu kann wahlweise das Ausgangssignal des Hochfrequenzgenerators oder das Ausgangssignal eines Hilfsgenerators herangezogen werden.

Abhängig von der Beschaffenheit des Gewebes sind unterschiedliche Generatoreinstellungen für ein schnelles Anschneiden notwendig. Daher besteht eine vorteilhafte Ausführung darin, daß der Sollwertgeber für den Fall, daß zu Beginn des Schnittes die Gewebeimpedanz an der Sonde niederohmig ist einen hohen Generatorstrom vorgibt. Eine andere vorteilhafte Ausführung besteht darin, daß der Sollwertgeber für den Fall, daß zu Beginn des Schnittes die Gewebeimpedanz an der Sonde hochohmig ist eine hohe Generatorspannung vorgibt.

Durch rechtzeitiges Detektieren unterschiedlicher Materialien, insbesondere metallischer Teile in der Nähe der Schneidelektrode kann die Leistung auf einen ungefährlichen Wert abgesenkt werden, bei dem Schneiden nicht mehr möglich ist und keine Koagulationsgefahr besteht. Wahlweise oder zusätzlich kann auch der Operateur gewarnt werden.

Dazu besitzt der Hochfrequenzgenerator eine Anzeigevorrichtung, die das vom Lichtbogen beeinflußte Spektrum der Leistung am Ausgang des Generators oder eine davon abhängige Größe wie Strom oder Spannung in mindestens zwei unterschiedlichen Frequenzbereichen vergleicht. Entsprechend dem Ergebnis des Vergleichs wird ein Sollwert abgegeben, der die Leistung des Generators beeinflußt. Hier werden zur Charakterisierung unterschiedlicher Materialien Unterschiede in deren Elektrophysikalischen Eigenschaften verwendet. Diese wirken sich stark auf den Lichtbogen aus, der beim Schneiden in der Hochfrequenzchirurgie zwischen Schneidelektrode und Gewebe entsteht. Dieser überbrückt die durch das Verdampfen von Zellflüssigkeit entstandene Dampfschicht zwischen Elektrode und Gewebe. Dieser Lichtbogen brennt nicht gleichmäßig auf der ganzen Oberfläche der Schneidelektrode. Er wird, eine ausreichend hohe Spannung vorausgesetzt, dort zünden, wo die Dampfschicht am dünnsten ist. Die durch den Lichtbogen hervorgerufene starke Energiekonzentration sorgt für ein rasches Verdampfen der Zellen an der Übertrittsstelle. Hier bildet sich nun eine stärkere Dampfschicht und der Lichtbogen wandert zu einer anderen Stelle, mit geringerem Isolationsabstand.

Charakteristisch für einen typischen Schneidevorgang sind die unterschiedlichen Materialien von Schneidewerkzeug und Schneidegut. Das Prinzip wird im Folgenden beispielhaft für den in der Praxis häufig vorkommenden Fall eines Gewebeschnittes mit einer Metallelektrode erklärt. Metall und Gewebe besitzen unterschiedliche physikalische Eigenschaften wie die Austrittsarbeit der Elektronen. Verstärkt wird dieser Effekt durch die unterschiedlichen Temperaturen der Materialien. So wird das Gewebe wegen dem Verdampfen der darin enthaltenen Zellflüssigkeit zunächst keine Temperaturen über der Siedetemperatur der Zellflüssigkeit annehmen. Damit sind Zünd- und Brennspannungen des Lichtbogens unterschiedlich je nachdem ob momentan das Metall das negativere Potential besitzt oder das Gewebe das negativere Potential aufweist. Beim Metall als Kathode ist die Zündspannung niedriger als beim Gewebe. Damit setzt beim Anlegen einer hochfrequenten Wechselspannung der Stromfluß je nach Polarität zu unterschiedlichen Zeitpunkten ein. Diese Unsymmetrie kann durch eine spektrale Auswertung gemessen werden. Je nach der Kombination von Schneidelektrode und zu schneidendem Material stellt sich daher eine charakteristische spektrale Leistungsverteilung ein.

Befindet sich nun ein Gebilde aus einem anderem Material so nahe an der Schneidelektrode, daß der Lichtbogen an dieses überspringt, so ändert sich durch die unterschiedlichen elektrischen Eigenschaften der Materialien die Symmetrie des Stromflußes und damit auch die spektrale Leistungsverteilung im Ausgangssignal des Generators. Eine Auswertung der unterschiedlichen spektralen Leistungsverteilung ist möglich durch den Vergleich von mindestens zwei nicht identischen Frequenzbereichen.

Durch die Auswertung der spektralen Leistungsverteilung können unterschiedliche Materialien differenziert weden. Ein typisches Beispiel ist die Unterscheidung zwischen Gewebe und metallisch leitenden Gebilden. Durch eine detailliertere Auswertung können auch mehrere Gewebearten voneinander unterschieden werden. Durch die Kenntnis des Materials ist eine Anpassung der Generatorleistung an die Verhältnisse am Operationsort möglich. So kann der Generator unmittelbar vor einer Berührung mit metallisch leitenden Gebilden abgeschaltet werden um Koagulationen des umliegenden Gewebes zu vermeiden.
Durch die Gewebedifferenzierung kann z.B. bei einer Prostataresektion nur noch das Adenomgewebe abgetragen werden. Schnitte in die Kapsel, die zu einer Perforation führen würden können verhindert werden.

Im Folgenden wird eine besonders vorteilhafte Ausführungsform beschrieben. Bei Materialien mit ähnlichen elektrischen Eigenschaften treten durch den Lichtbogen überwiegend ungeradzahlige Harmonische der Generatorfrequenz auf, während bei unterschiedlichen elektrischen Eigenschaften die geradzahlige Harmonischen der Generatorfrequenz überwiegen. Bei gleichen Materialien treten nur ungeradzahlige Harmonische der Generatorfrequenz auf. Unter dem Begriff "Harmonische" werden werden hier die Vielfachen der Generatorfrequenz verstanden, einschließlich der Harmonischen 0-ter Ordnung, die dem Gleichanteil ( f = 0) entspricht. Daher besteht eine besonders vorteilhafte Ausführungsform darin, daß in der Anzeigeeinrichtung zwei Filter zur Auswertung der spektralen Anteile des Generatorsignals vorhanden sind und das erste Filter überwiegend die spektrale Leistung bei einer oder mehreren der ungeradzahligen Harmonischen der Grundfrequenz des Generators f ₀ erfaßt und das zweite Filter überwiegend die spektrale Leistung bei einer oder mehreren geradzahligen Harmonischen der Grundfrequenz des Generators f ₀ erfaßt. Diese Ausführungsform läßt sich besonders gut zur Erkennung von metallischen Gebilden im Gewebe verwenden.

Für die Erkennung unterschiedlicher Materialien kann dem Generatorsignal mit der Frequenz f ₀ ein Hilfsoszillatorsignal kleiner Leistung mit der Frequenz f _{H} additiv überlagert werden. Dabei entstehen durch die Nichtlinearität des Lichtbogens Verzerrungen und damit Mischprodukte höherer Ordnung. Bei ähnlichen Materialien entstehen überwiegend Mischprodukte zweiter Ordnung mit den Frequenzen 2 f ₀, 2 f _{H} , f ₀ + f _{H} , f ₀ - f _{H} , während bei unterschiedlichen Materialien die Mischprodukte dritter Ordnung mit den Frequenzen 3 f ₀, 3 f _{H}, 2 f ₀ + f _{H}, f ₀ + 2 f_{H}, 2 f ₀ - f _{H} , f ₀ - 2 f _{H} , f _{H} + f ₀ - f ₀ entstehen.

Daher besteht eine weitere vorteilhafte Ausführungsform darin, daß ein Hilfsoszillatorsignal kleiner Leistung dem Generatorsignal additiv überlagert wird. Bei der Auswertung werden durch das erste Filter eines oder mehrere der Mischprodukte zweiter Ordnung und durch das zweite Filter eines oder mehrere der Mischprodukte dritter Ordnung erfaßt. Solche Verfahren sind besonders vorteilhaft, wenn der Generator nicht ausreichend oberwellenfrei ist, oder wenn die Generatorfrequenz nicht ausreichend stabil ist, so daß das Ausfiltern der Harmonischen einen hohen Aufwand erfordern würde.

Eine besonders vorteilhafte Ausführungsform zu Nutzung der gewonnenen Information über Unterschiede im Gewebe oder anderen Materialien besteht darin, diese zur Regelung der Generatorleistung oder zu einer starken Reduktion der Generatorleistung zu verwenden, so daß kein Schnitt und/oder keine Koagulation des Gewebes möglich ist.

Eine weitere Ausführungsform ist eine Warneinrichtung, die den Nutzer des Hochfrequenzchirurgiegenerators warnt. Der Nutzer kann dann gefährliche Zustände erkennen und z.B. die Schnittführung anders wählen, den Generator abschalten, fehlerhafte Operationsinstrumente auswechseln.

Eine Reduktion der Generatorleistung wird vorteilhafterweise über ein Zeitglied gesteuert, so daß nach dem Erkennen eines bestimmten Gewebes oder anderen Materials die Leistung des Generators für eine vorbestimmte Zeit abgesenkt bleibt. Dadurch wird vermieden, daß durch zu häufiges Erhöhen der Generatorleistung dem Gewebe eine unnötig hohe Leistung zugeführt wird, denn nach dem Absenken der Generatorleistung tritt definitionsgemäß kein Lichtbogen mehr auf und die Anzeigeeinrichtung kann das Gewebe oder Material nicht mehr erkennen. Ohne Zeitglied würde damit unmittelbar nach der Leistungsabsenkung die Leistung wieder erhöht werden. Die Zeit des Zeitgliedes kann eine Halbwelle dauern, sie kann aber auch wesentlich länger sein. Für spezielle Anwendungen kann es sinnvoll sein, eine Generatoraktivierung erst bei einem neuen Schnitt freizugeben.

Zusätzlich kann eine Überwachung der Gewebeimpedanz eingebaut werden. Sie erkennt Impedanzsprünge, wie sie z.B. beim Entfernen der Schneidelektrode auftreten und beendet dann das Zeitintervall des Zeitgebers.

Ein Hochfreqenzgenerator für die Hochfrequenzchirurgie, der zur Minimierung der Störung anderer Geräte bzw. der Reizung bestimmter Muskeln und Nerven in seiner Ausgangsleistung oder einer davon abhängigen elektrischen Größe steuerbar ist besitzt eine Austasteinrichtung mit deren Hilfe die Ausgangsleistung des Generators reduziert werden kann.
Der Generator kann entsprechend dem Anwendungsfall keine, eine oder mehrere Regelungen seiner Ausgangsgrößen besitzen. Die Austasteinrichtung wird von einer Meßeinrichtung zur Feststellung zu schützender Zeiten gesteuert. Dadurch wird zu diesen Zeiten die Ausgangsleistung des Generators so verringert oder auch vollständig abgeschaltet, so daß die durch das Generatorsignal direkt oder indirekt verursachten Störungen vernachlässigt werden können. Darunter fallen auch Störungen, die durch Modulation und / oder nichtlineare Effekte außerhalb des Generators auftreten. Eine besondere zeitabhängige Nichtlinearität stellt hier der Lichtbogen dar. So können während der zu schützenden Zeiten keine Störungen anderer elektronischer Geräte oder Muskel- und Nervenreizungen auftreten. Die Zeitintervalle, in denen die Generatorleistung reduziert wird, können länger sein, oder auch eine zeitliche Verschiebung zu den von der Meßeinrichtung zur Feststellung zu schützender Zeiten ermittelten Zeitintervallen aufweisen. Dies kann z.B. notwendig sein, um ein Entladen des nach den Sicherheitsvorschriften des VDE im Generatorausgang vorhandenen Serienkondensators zu ermöglichen. Dieser kann während eines Schnittes durch den von der Nichtlinearität des Lichtbogens verursachten Gleichanteil aufgeladen werden. Am Beginn des zu schützenden Zeitintervalles muß auch die Ladung aus diesem Kondensator rechtzeitig abgeflossen sein, da sonst durch den Entladestrom ebenfalls Muskel- und Nervenreizungen verursacht werden können. Die Zeitintervalle sollen so groß gewählt sein, daß sich die ursprüngliche operative Zielsetzung nicht ändert. So soll durch die Modulation eines primär zum Schneiden ausgelegten Generatorsignals keine überwiegend koagulierende Wirkung entstehen. Weiterhin besitzt der Generator eine Einrichtung zur Festlegung der inneren Zustände nach den Zeitintervallen in denen die Generatorleistung verringert wird. Damit können für die internen Schaltungsteile des Generators und seiner Steuer- und Regeleinrichtungen Sollwerte und Einstellungen für das folgende Zeitintervall vorgegeben werden. Diese können fest voreingestellt oder variabel sein und eine Anpassung an das Operationsziel ermöglichen.

Charakteristisch für Regler, wie sie in HF-Chirurgiegeneratoren eingesetzt werden, ist ihre Zeitkonstante, die wesentlich größer als die Periodendauer der Generatorfrequenz sein muß. Bei einem Schnitt mit periodischen Unterbrechungen müßten diese Regler zu Beginn eines jeden Schnittintervalles einschwingen. Dies kann zu Koagulationen führen. Damit verschlechtert sich die Schnittqualität. Kennzeichnend für einen Schnitt mit solchen kurzen Unterbrechungen ist jedoch, daß sich die physikalischen Eigenschaften des Gewebes am Operationsort während dieser kurzen Zeitintervalle nur unwesentlich ändern. Daher wäre eine Fortsetzung des Schnittes mit den gleichen Parametern der Generatoreinstellung möglich. Hierzu werden nicht nur die Vorgabewerte für die Parametereinstellungen gespeichert, sondern auch die internen Zustände der Regler festgehalten.

Eine besonders vorteilhafte Ausführung besteht darin, daß der Generator eine Speichereinheit zur Speicherung aller wichtigen Parameter, insbesondere der Zustände der internen Regler besitzt. Als Beispiel wäre bei analogen Reglern eine Realisierung mit analogspeichern und bei digitalen Reglern eine Realisierung mit Digitalspeichern möglich. Ein Speichern der Werte erfolgt jeweils am Ende des Schneidintervalles zu Beginn der Pause.
Zu Beginn des nächsten Zeitintervalles am Ende der Pause werden diese Werte abgerufen und zur Initialisierung der Regler verwendet. Damit kann ein schnelles Einschwingen der Regler und damit ein schnelles und gewebeschonendes Anschneiden erreicht werden.

Eine weitere vorteilhafte Ausführungsform besteht darin, daß ein Sollwertgeber vorhanden ist, der nach dem Zeitintervall, in dem die Generatorleistung reduziert wird Sollwerte für die Regelung der Ausgangsleistung vorgibt. Diese Sollwerte können mittleren Generatorleistungen bei durchschnittlichen Operationen entsprechen. Damit wird zum Ende der zu schützenden Zeitintervalle ein definiertes Anschneideverhalten des Generators erreicht, da hier die Regler nicht von einem Nullzustand sondern von einem den tatsächlichen Schnittbedingungen sehr naheliegenden Anfangszustand ausgehen.

Bei länger ausgedehnten zu schützenden Zeitintervallen können sich die Bedingungen am Operationsort allmählich verändern. Daher besteht eine weiter vorteilhafte Ausführungsform darin, daß wahlweise auch beim Wiederanschneiden ein Sollwert für eine höhere Leistung vorgegeben werden kann. Damit kann dem Gewebe in kürzerer Zeit die zum Schnittbeginn notwendige Energie zugeführt werden. Sobald die Meßeinrichtung zur Feststellung des Schneidens einen Schnittbeginn detektiert, wird auf eine niedrigere, zum Schneiden gerade noch ausreichende Leistung zurückgeschaltet. Dadurch werden Effekte des Auskühlens oder einer Flüssigkeitsansammlung im Gewebe kompensiert und ein zügiges Anschneiden mit nur geringer Koagulation wird erreicht.

Eine weitere vorteilhafte Ausführung besteht darin, daß die Meßeinrichtung zur Feststellung zu schützender Zeiten die zu schützenden Zeitintervalle aufgrund des Herzrhytmus des Patienten ermittelt. Dieser kann z.B. von EKG - Signalen abgeleitet werden. Besonders bei Operationen in der Nähe des Herzens oder bei Trägern von Herzschrittmachern kann damit die Leistungsabgabe des Generators auf die Zeiten beschränkt werden, in denen der Herzmuskel durch elektrische Felder nicht erregt werden kann. Damit kann die Gefahr von Herzkammerflimmern wesentlich verringert werden.

Häufig werden bei Hochfrequenzchirurgischen Operationen andere Geräte betrieben, die eine Datenübertragung mit hoher Datenübertragungsrate notwendig machen. So werden Daten zwischen Rechnern oder anderen Geräten in digitaler oder analoger Form übertragen. Dies können Meßdaten oder auch Werte sein, die auf einem Bildschirm dargestellt werden. Dazu können diese Daten von bildgebenden Systemen aller Art wie Ultraschallgeräten oder Röntgengeräten abgeleitet werden. Diese Daten werden in Computersystemen oder Videoprozessoren weiterverarbeitet und aufgezeichnet. Zu bestimmte Zeiten kann eine Verfälschung dieser Daten besonders kritische Auswirkungen haben und deren Informationsgehalt wesentlich verfälschen. Daher besteht eine vorteilhafte Ausführungsform darin, daß die Meßeinrichtung zur Feststellung zu schützender Zeiten besonders diejenigen Zeitintervalle ermittelt, in denen die höchste Störanfälligkeit dieser übertragenen Daten gegeben ist. Dies ist z.B. der Fall, wenn die Daten für eine Offline-Auswertung von einem Rechner auf das Speichermedium übertragen werden.

### Kurze Beschreibung der Zeichnungen

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1 :: Prinzipschaltbild des Hochfrequenzchirurgiegenerators zum Schneiden von Geweben,
- Fig. 2 :: Beispielhafte Darstellung des Impedanzanstieges am Schnittbeginn,
- Fig. 3 :: Prinzipschaltbild des Hochfrequenzchirurgiegenerators zur gewebe- bzw. materialabhängigen Operationsdurchführung,
- Fig. 4 :: Prinzipschaltbild des Hochfrequenzchirurgiegenerators mit Zusatzeinrichtungen für vorteilhafte Ausführungsformen,
- Fig. 5 :: Beispielhafte Darstellung der spektralen Verteilung der Generatorleistung und beispielhafte Filterkurven,
- Fig. 6 :: Prinzipschaltbild des Hochfrequenzchirurgiegenerators mit minimalen negativen Rückwirkungen auf das Umfeld,
- Fig. 7 :: Beispielhafte Darstellung der Synchronisation auf ein EKG-Signal.

### Beschreibung eines Ausführungsbeispiels

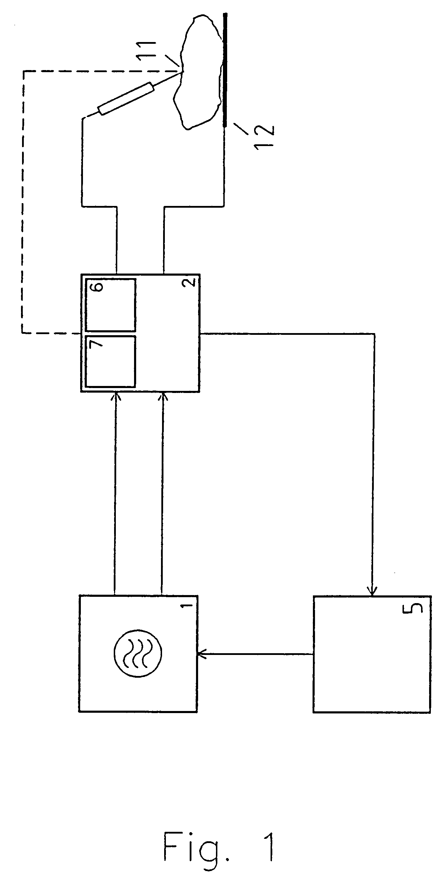

In Fig. 1 ist das Prinzipschaltbild eines Hochfrequenzchirurgiegenerators dargestellt, mit dem beim Schneiden von Geweben unerwünschte Nebenwirkungen weitgehend reduziert werden. Der Hochfrequenzgenerator 1 für die Hochfrequenzchirurgie besitzt Regeleinrichtungen zur Einstellung der momentanen elektrischen Ausgangsgrößen wie z.B. Strom, Spannung und Leistung. Der zum Schneiden benötigte Hochfrequenzstrom wird über die Sonde 11 in das Gewebe eingeleitet und tritt durch die Neutralelektrode 12 wieder aus dem Patienten aus. Die Kontaktfläche der Sonde 11 ist wesentlich kleiner als die Fläche der Neutralelektrode 12, so daß die thermischen Effekte, die der Hochfrequenzchirurgie zugrunde liegen, an der Sonde 11 auftreten. Eine Einrichtung 2 dient zur unmittelbaren und/oder mittelbaren Feststellung des Schneidens. Diese Einrichtung kann einen Meßgeber in unmittelbarer Nähe der Sonde enthalten. Außerdem kann sie eine Auswerteeinrichtung zur Auswertung der elektrischen Ausgangsgrößen des Generators enthalten. Wahlweise kann die Einrichtung zur unmittelbaren und/oder mittelbaren Feststellung des Schneidens einen Prüfsignalgenerator 6 enthalten, dessen Prüfsignal der Sonde zugeführt und mit Hilfe der Meßeinrichtung 2 gemessen wird. Die Meßeinrichtung kann einen Grenzwertgeber 7 besitzen, der die Schwelle für den Zustand "Schneiden" festlegt.

Weiterhin enthält die Vorrichtung einen Sollwertgeber 5, der entsprechend dem Ausgangssignal der Meßeinrichtung den Sollwert für die Ausgangsleistung oder einer anderen dazu proportionalen elektrischen Größe des des Hochfrequenzgenerators vorgibt.

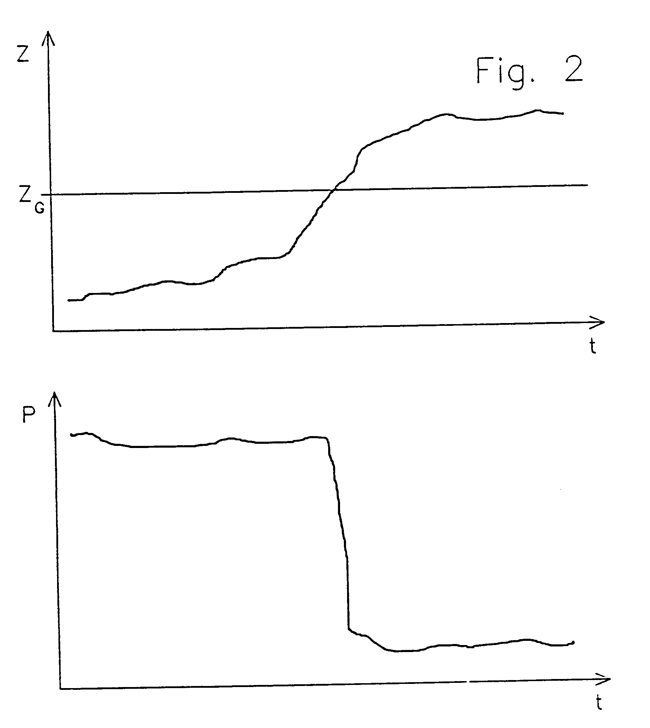

In Fig. 2 ist der Verlauf der Gewebeimpedanz am Beginn eines typischen Schnittes in Magen- oder Darmwand dargestellt, wie er in vielen Messungen ermittelt wurde. Zunächst ist wegen der leitfähigen Flüssigkeit auf der Oberfläche die Gewebeimpedanz sehr niedrig.
Nachden ausreichend Energie zugeführt wurde um diese Flüssigkeitsschicht zu verdampfen, steigt die Impedanz stark an. Nun ist erst ein Schneiden möglich. Damit nur eine geringfügige Anschneideverzögerung auftritt ist es notwendig, die zum Verdampfen der Flüssigkeit benötigte Energie möglichst schnell zuzuführen. Daher sollte zu Beginn eines Schnittes die Generatorausgangsleistung möglichst hoch sein. Nachdem der Schnittbeginn z.B. durch Auswertung der Gewebeimpedanz und deren Vergleich mit einer Schwelle Z _{G} festgestellt wurde, kann die Generatorleistung auf einen üblicherweise zum Schneiden ausreichenden Wert abgesenkt werden.

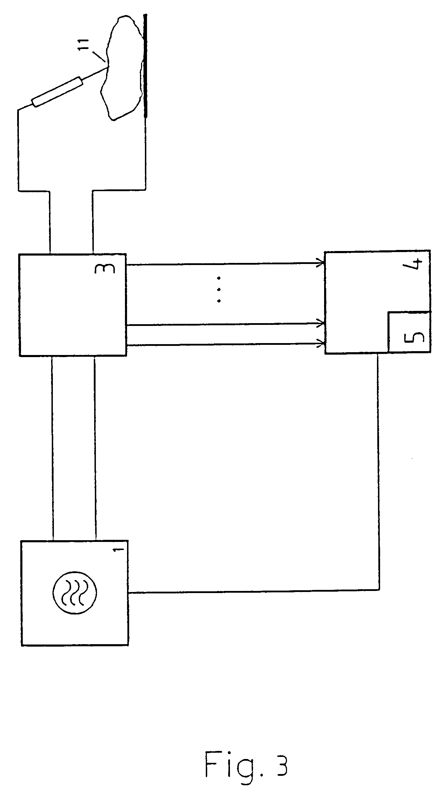

In Fig. 3 ist das Prinzipschaltbild eines Hochfrequenzchirurgiegenerators dargestellt, bei dem die Leistung, beispielsweise beim Metallkontakt, auf einen ungefährlichen Wert abgesenkt werden kann. Der Hochfrequenzgenerator 1 besitzt eine Vorrichtung zur mittelbaren und/oder unmittelbaren Einstellung der maximalen Ausgangsleistung. Zwischen dem Hochfrequenzchirurgiegenerator 1 und der Schneidelektrode 11 befindet sich eine Anzeigeeinrichtung 3 zur Anzeige der Leistung oder einer davon abhängigen Größe in zwei oder mehreren unterschiedlichen Frequenzbereichen mit elektrischen Signalen. Die elektrischen Ausgangssignale dieser Anzeigeeinrichtung 3 werden in eine Auswerteschaltung 4 geführt. Diese enthält einen Sollwertgeber 5, welcher die Sollwerte zur Einstellung des Hochfrequenzgenerators 1 vorgibt. Die Sollwertvorgabe erfolgt in Abhängigkeit vom Verhältnis der Ausgangssignale der Anzeigevorrichtung.

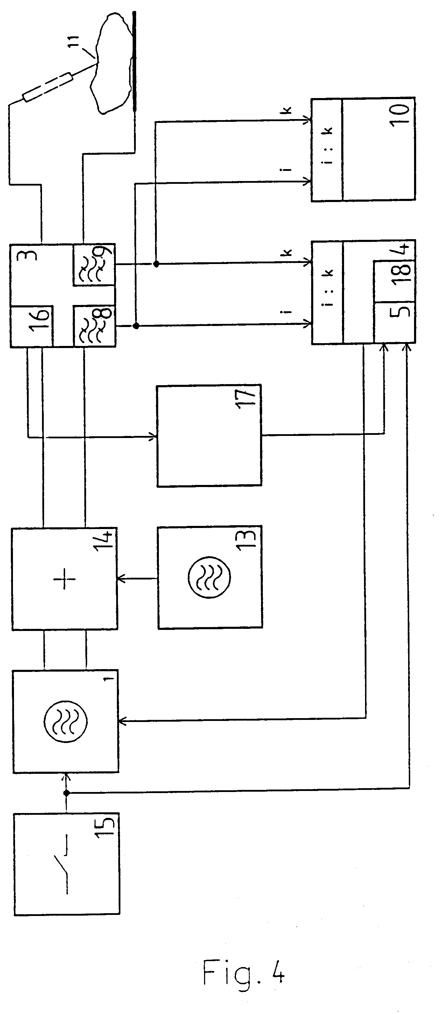

Mit Fig. 4 sollen einige vorteilhafte Ausführungsformen verdeutlicht werden. Es sind alle Elemente aus Fig. 1 enthalten. Diese sind im vorhergehenden Abschnitt beschrieben. Zur Selektion zweier unterschiedlicher Frequenzbereiche besitzt die Anzeigevorrichtung 3 zwei Filter 8 und 9. Die beiden elektrischen Ausgangssignale (i), (k) der Anzeigevorrichtung 3 können auch wahlweise an eine Warneinrichtung 10 zur Warnung des Operateurs geführt werden. Diese Warneinrichtung wird betätigt, wenn das Verhältnis der beiden Ausgangssignale der Anzeigevorrichtung 3 einen bestimmten Wert übersteigt. Weiterhin ist ein zusätzlicher Hilfsoszillator 13 eingezeichnet, dessen Signal kleiner Leistung über das Koppelelement 14 additiv zum Ausgangssignal des Hochfrequenzgenerators überlagert wird. Der Schalter 15 dient zum Aktivieren des Hochfrequenzgenerators. Außerdem beendet er das Zeitintervall zur Abschaltung des Generators, das der Zeitgeber in der Auswerteschaltung 4 erzeugt. Weiterhin befindet sich in der Anzeigevorrichtung 3 eine Vorrichtung 16 zur Ermittlung der Impedanz des Gewebes an der Schneidelektrode 11. Diese Impedanz wird in einer Impedanzauswerteschaltung 17 ausgewertet und ebenfalls zur Beendigung des Zeitintervalls zur Abschaltung des Generators herangezogen.

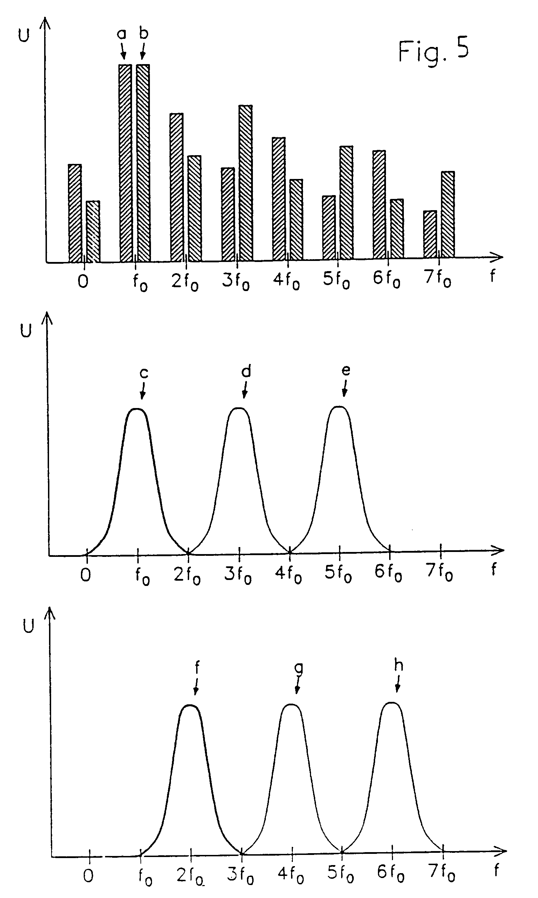

In Fig. 5 sind oben beispielhaft die Spektrallinien zweier unterschiedlicher Spektren dargestellt, wie sie bei einem schmalbandigen Hochfrequenzgenerator auftreten. Dabei ist eine Spannung U als stellvertretende Größe für eine der Ausgangsgrößen des Generators aufgetragen. Die Säulenpaare in der Grafik zeigen die Amplituden bei den Vielfachen der Generatorfrequenz f ₀. Die linken Säulen (a) dieser Paare verdeutlichen das Spektrum bei großen Unterschieden in den physikalischen Eigenschaften zwischen Elektrodenmaterial und dem zu schneidenden Material. Besitzen beide Materialien ähnliche physikalischen Eigenschaften, so kann sich ein Spektrum ergeben, wie es die rechten Säulen (b) darstellen. Beide Spektren sind auf eine gleiche Amplitude der Grundwelle ( f ₀) normiert. Die mittlere Abbildung in Fig. 5 zeigt beispielhaft eine mögliche spektrale Durchlaßcharakteristik, wie sie das zweite Filter 9 aus Anspruch 8 besitzen könnte. Zur Auswertung genügt die Selektion eines der drei beispielhaft dargestellten Frequenzbereiche (c), (d) oder (e), ebenso können auch mehrere solcher Frequenzbereiche zusammengefaßt werden. Die untere Abbildung in Fig. 5 zeigt beispielhaft eine mögliche spektrale Durchlaßcharakteristik, wie sie das erste Filter 8 aus Anspruch 8 besitzen soll. Zur Auswertung genügt die Selektion eines der drei beispielhaft dargestellten Frequenzbereiche (f), (g) oder (h), ebenso können auch hier mehrere Frequenzbereiche zusammengefaßt werden.

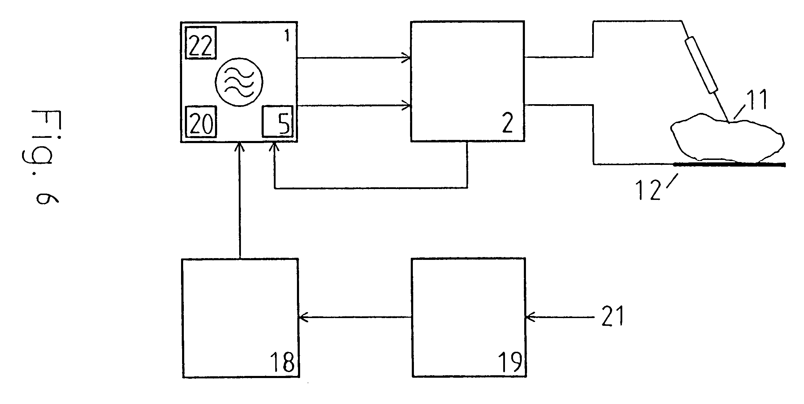

In Fig. 6 ist das Prinzipschaltbild des Hochfrequenzchirurgiegenerators nach der Erfindung dargestellt. Der Hochfrequenzgenerator 1 für die Hochfrequenzchirurgie ist in seiner Ausgangsleistung steuerbar. Der Hochfrequenzstrom wird über die Sonde 11 in den Patienten eingespeist. Der Rückfluß des hochfrequenten Stromes erfolgt über die Neutralelektrode 12. Eine Austasteinrichtung 18 steuert den Generator 1 so, daß in bestimmten Zeitintervallen die Ausgangsleistung reduziert wird. Diese Zeitintervalle werden von einer Einrichtung 19 zur Feststellung der vor Generatorleistung zu schützenden Zeiten vorgegeben. Dieser Einrichtung 19 wird ein Signal 21 zugeführt, das den Herzrhytmus des Patienten oder andere biologische Signale wiedergibt. Wahlweise kann der Einrichtung 19 auch ein Signal zugeführt werden, das von Datenverarbeitungsanlagen stammt und Zeiten angibt, in denen eine Störung die Wirkung der Anlage besonders beeinträchtigt.
Die Vorrichtung 22 dient zur Festlegung der inneren Zustände des Generators nach den Zeitintervallen, in denen die Generatorleistung verringert wird. Ein Sollwertgeber 5 im Generator gibt Sollwerte für die Einstellung des Generators vor. Eine Meßeinrichtung 2 zur Feststellung des Schneidens erkennt den Schnittbeginn und steuert damit den Sollwertgeber 5. Eine Speichereinheit 20 im Generator erlaubt eine Speicherung der letzten internen Zustände des Generators, um eine Fortsetzung des Schnittes mit den gleichen Parametern zu gewährleisten.

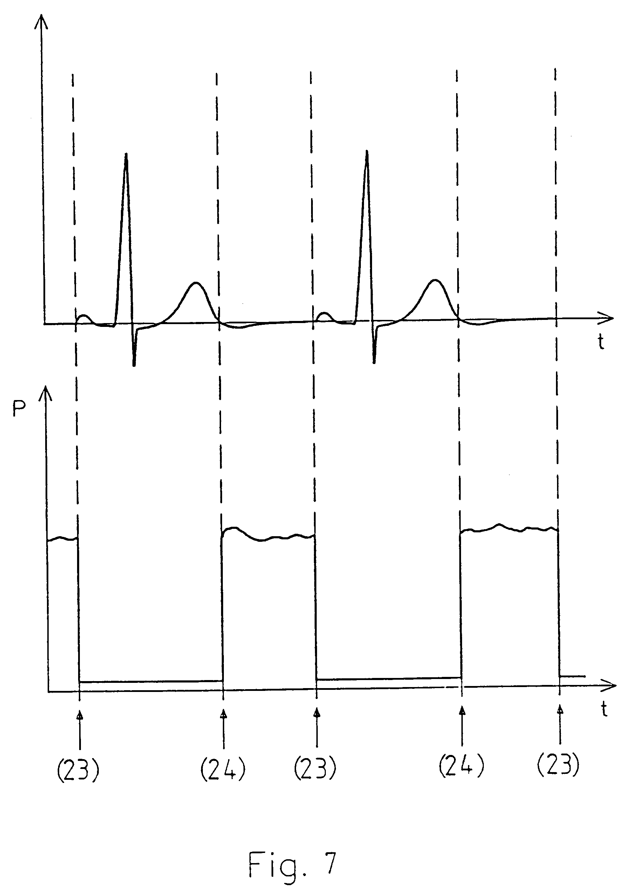

In Fig. 7 ist beispielhaft die Austastung gesteuert durch ein EKG-Signal dargestellt.
In dem oberen Diagramm ist das EKG-Signal über der Zeit aufgetragen. Im unteren Diagramm ist schematisiert der zeitliche Verlauf der Generatorleistung im gleichen Zeitmaßstab dargestellt. Der Generator wird hier nur zu den Zeiten aktiviert, in denen eine Reizung des Herzmuskels oder eines Herzschrittmachers nicht möglich ist. Die Abschaltung des Generators erfolgt zu den Zeitpunkten 23, zu denen auch die Speicherung in der Speichereinheit 20 getriggert wird. Eine Aktivierung des Generators und ein Abrufen der Speicherzustände erfolgt zu den Zeitpunkten 24.

## Patentansprüche

1. Hochfrequenz-Chirurgiegenerator (1) zum Schneiden von Gewebe mit
- einer Sonde (11) zur Einleitung des HF-Stroms des Chirurgiegenerators in das Gewebe,
- wenigstens einer Regeleinrichtung für eine der momentanen elektrischen Ausgangsgrößen des Chirurgiegenerators, und
- einem Sollwertgeber (5), der einen Sollwert für die von der Regeleinrichtung geregelte Ausgangsgröße des Chirurgiegenerators vorgibt,
**dadurch gekennzeichnet**, daß eine Meßeinrichtung (2) vorgesehen ist, die erfaßt, ob die Sonde (11) das Gewebe trennend schneidet, und
daß das Ausgangssignal der Meßeinrichtung (2) an dem Sollwertgeber zur Sollwert-Einstellung anliegt, so daß der Sollwertgeber zu Beginn des Schneidvorgangs zunächst einen Sollwert abgibt, der eine für das Anschneiden besonders geeignete Einstellung der Ausgangsgröße bewirkt, und nachdem die Meßeinrichtung (2) ein gewebetrennendes Schneiden der Sonde (11) erfaßt, diesen Sollwert zur Ausführung eines normalen Schnitts ändert.

2. Chirurgiegenerator nach Anspruch 1,
**dadurch gekennzeichnet**, daß ein Grenzwertgeber (7) vorgesehen ist, der den von der Meßeinrichtung erfaßten Wert mit einem Grenzwert vergleicht, und
daß der Sollwertgeber beim Überschreiten dieses Grenzwertes den Sollwert auf den normalen, dem gewünschten Operationsziel entsprechenden Wert einstellt.

3. Chirurgiegenerator nach Anspruch 1 oder 2,
**dadurch gekennzeichnet**, daß die Meßeinrichtung den von der Sonde im Gewebe zurückgelegten Weg erfaßt.

4. Chirurgiegenerator nach Anspruch 1 oder 2,
**dadurch gekennzeichnet**, daß die Meßeinrichtung die Impedanz zwischen der Sonde (11) und einer Neutralelektrode (12) erfaßt.

5. Chirurgiegenerator nach Anspruch 4,
**dadurch gekennzeichnet**, daß der Sollwertgeber bei einer niedrigen Impedanz zwischen Sonde und Neutralelektrode einen hohen Generatorstrom solange vorgibt, bis das Ausgangssignal der Meßeinrichtung "Schneiden" angibt.

6. Chirurgiegenerator nach Anspruch 1 oder 2,
**dadurch gekennzeichnet**, daß die Meßeinrichtung (2) den an der Sonde (11) auftretenden Lichtbogen erfaßt.

## Claims

1. A surgical high-frequency generator (1) for cutting tissue having
- a probe (11) for introducing the high-frequency current into the tissue,
- at least one control device for one of the momentary electric output values of said surgical generator, and
- a desired-value transmitter (5) which provides the desired value for the output value of said surgical generator regulated by said control device,
**characterized by** a measurement device (2) being provided which detects whether said probe (11) is severing the tissue, and
by the output signal of said measurement device (2) being applied to said desired-value transmitter for setting the desired value, so that upon commencement of the cutting procedure said desired-value transmitter first provides a desired value which results in a setting of the output value particularly suited for incision and after said measurement device (2) has detected severing of the tisssue by said probe (11), changes said desired value for carrying out a normual cut.

2. A surigcal generator according to claim 1,
**characterized by** a limit-value transmitter (7) being provided which compares the value detected by said measurement device with a limit value, and
by upon exceeding said value limit, said desired-value transmitter setting the desired value to the normal value suited for the desired surgical goal.

3. A surgical generator according to claim 1 or 2,
**characterized by** said measurement device detecting the path covered by said probe in the tissue.

4. A surgical generator according to claim 1 or 2,
**characterized by** said measurement device detecting the impedance between said probe (11) and a neutral electrode (12).

5. A surgical generator according to claim 4,
**characterized by** said desired-value transmitter providing a high generator current if there is low impedance between said probe and said neutral electrode until said output signal of said measurement device says "cutting".

6. A surgical generator according to claim 1 or 2,
**characterized by** said measurement device (2) detecting the electric arc occuring at said probe (11).

## Revendications

1. Générateur chirurgical à haute fréquence (1) pour le découpage d'un tissu, comprenant
- une sonde (11) pour introduire dans le tissu le courant à haute fréquence du générateur chirurgical,
- au moins un dispositif de régulation pour l'une des grandeurs électriques instantanées de sortie du générateur chirurgical, et
- un générateur de valeur de consigne (5), qui prédétermine une valeur de consigne pour la grandeur de sortie du générateur chirurgical, réglée par le dispositif de régulation,
caractérisé en ce qu'il est prévu un dispositif de mesure (2) qui détecte si la sonde (11) coupe le tissu en le séparant, et
que le signal de sortie du dispositif de mesure (2) est appliqué au générateur de valeur de consigne pour le réglage de la valeur de consigne de telle sorte que le générateur de valeur de consigne délivre tout d'abord, au début du processus de coupe, une valeur de consigne, qui réalise un réglage, particulièrement approprié pour le démarrage de l'opération de coupe, de la grandeur de sortie et, après que le dispositif de mesure (2) détecte le fait que la sonde (11) effectue une coupe séparant le tissu, modifie cette valeur de consigne pour l'exécution d'une coupe normale.

2. Générateur chirurgical selon la revendication 1, caractérisé en ce qu'il est prévu un générateur de valeur limite (7), qui compare la valeur détectée par le dispositif de mesure à une valeur limite, et que lors du dépassement de cette valeur limite, le générateur de valeur de consigne règle la valeur de consigne sur la valeur normale, qui correspond au but désigné de l'opération.

3. Générateur chirurgical selon la revendication 1 ou 2, caractérisé en ce que le dispositif de mesure détecte le trajet parcouru par la sonde dans le tissu.

4. Générateur chirurgical selon la revendication 1 ou 2, caractérisé en ce que le dispositif de mesure détecte l'impédance entre la sonde (11) et une électrode neutre (12).

5. Générateur chirurgical selon la revendication 4, caractérisé en ce que dans le cas d'une faible impédance entre la sonde et l'électrode neutre, le générateur de valeur de consigne prédétermine un courant intense du générateur tant qu'un signal de consigne du dispositif de mesure indique "coupe".

6. Générateur chirurgical selon la revendication 1 ou 2, caractérisé en ce que le dispositif de mesure (2) détecte l'arc électrique apparaissant au niveau de la sonde (11).
